(19) **Europäisches Patentamt / European Patent Office / Office européen des brevets**

(11) **EP 3 730 685 B1**

(12) **EUROPEAN PATENT SPECIFICATION**

(45) Date of publication and mention
of the grant of the patent:
**12.06.2024 Bulletin 2024/24**

(21) Application number: **18890630.9**

(22) Date of filing: **18.12.2018**

(51) International Patent Classification (IPC):
*D04H 3/16* (2006.01)   *A61F 13/15* (2006.01)
*A61F 13/514* (2006.01)   *A61L 15/24* (2006.01)
*D04H 3/007* (2012.01)   *D04H 1/4382* (2012.01)
*D04H 1/544* (2012.01)   *D04H 1/56* (2006.01)
*D04H 3/016* (2012.01)

(52) Cooperative Patent Classification (CPC):
(C-Sets available)
**D04H 3/016; A61F 13/514; A61L 15/24;
D04H 1/43838; D04H 1/544; D04H 1/56;
D04H 3/007; D04H 3/16**   (Cont.)

(86) International application number:
**PCT/JP2018/046653**

(87) International publication number:
**WO 2019/124408 (27.06.2019 Gazette 2019/26)**

(54) **MELTBLOWN NON-WOVEN FABRIC**

SCHMELZGEBLASENER VLIESSTOFF

NON-TISSÉ OBTENU PAR FUSION-SOUFFLAGE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2017   JP 2017244951
16.11.2018   JP 2018215397**

(43) Date of publication of application:
**28.10.2020   Bulletin 2020/44**

(73) Proprietor: **Kao Corporation
Chuo-ku,
Tokyo 103-8210 (JP)**

(72) Inventors:
• **ITO, Tatsunori
Haga-gun, Tochigi 321-3497 (JP)**
• **SASE, Masakazu
Haga-gun, Tochigi 321-3497 (JP)**
• **NIITSU, Taichi
Haga-gun, Tochigi 321-3497 (JP)**

(74) Representative: **Vossius & Partner
Patentanwälte Rechtsanwälte mbB
Siebertstrasse 3
81675 München (DE)**

(56) References cited:
**EP-A1- 2 527 508       EP-A1- 3 255 188
JP-A- H0 860 515        JP-A- H05 179 554
JP-A- 2003 502 514      JP-A- 2009 062 667
JP-A- 2009 504 933      JP-A- 2010 185 154
JP-A- 2011 168 944      JP-A- 2013 506 062
JP-A- 2014 176 775      JP-A- 2016 521 778
US-A1- 2013 266 874**

**(Cont. next page)**

(52) Cooperative Patent Classification (CPC): (Cont.)

C-Sets
**A61L 15/24, C08L 23/12**

C-Sets
**A61L 15/24, C08L 23/12**

**Description**

Technical Field

The present invention relates to a meltblown non-woven fabric produced by meltblowing.

Background Art

Meltblown non-woven fabrics are non-woven fabrics produced by meltblowing and have been known to have a short inter-fiber distance and a high water resistance because fine fibers are densely entangled (WO 2012/102398 A) .

Meltblowing is a process including: a spinning step of blowing a molten thermoplastic resin composition through a die with multiple nozzles by using a high-temperature and high-speed air stream to draw the thermoplastic resin composition into a fiber; and a deposition step of depositing the resulting fibers on a collection surface to fuse one another, so that the process is suitable for producing fine fibers.

EP 2 527 508 A1 discloses a nonwoven fabric according to the preamble of claim 1. Other technology is shown in EP 3 255 188 A1 or US 2013/266874 A1.

Summary of Invention

The present invention provides a meltblown non-woven fabric as defined in independent claim 1.

In the invention, the meltblown non-woven fabric has a ratio of the rate of straight-running fibers in the first direction to the rate of straight-running fibers in the second direction (the rate of straight-running fibers in the first direction/the rate of straight-running fibers in the second direction) of from 1 to 2.5.

In an embodiment, the meltblown non-woven fabric has a water pressure resistance of from 100 mm $H_2O$ to 10000 mm $H_2O$. In an embodiment, the meltblown non-woven fabric has a water pressure resistance retention rate of 85% or higher when the meltblown non-woven fabric is deformed in the second direction.

In an embodiment, the meltblown non-woven fabric has a filling factor of 3% or more and/or 30% or less.

In an embodiment, the meltblown non-woven fabric has a formation index of 30 or more and/or 300 or less.

In an embodiment, the meltblown non-woven fabric has a ratio of the rate of straight-running fibers in the first direction to the rate of straight-running fibers in the second direction (the rate of straight-running fibers in the first direction/the rate of straight-running fibers in the second direction) of from 1 to 1.9.

In an embodiment, the meltblown non-woven fabric has a filling factor of 6% or more and/or 15% or less.

In an embodiment, the meltblown non-woven fabric has a formation index of 30 or more and/or 300 or less and optionally 200 or less.

In an embodiment, the fibers have a heat of fusion of 20 mJ/mg or more and/or 80 mJ/mg or less.

In addition, the invention provides a leakage prevention sheet having the meltblown non-woven fabric, as defined in independent claim 6.

In addition, the invention provides an absorption article as defined in independent claim 7 including:

a liquid permeating surface sheet that is arranged on a skin facing surface side;
a liquid leakage-preventing other surface sheet that is arranged on a skin non-facing surface side; and
an absorbent that is interposed between the sheets, wherein
the other surface sheet is the above leakage prevention sheet.

In addition, the invention provides a method of producing a meltblown non-woven fabric as defined in independent claim 8, including a spinning step of discharging a molten thermoplastic resin composition from a nozzle to produce a fiber by using an air stream.

In the production method according to the invention, a meltblown non-woven fabric with an average fiber diameter of 4 um or less is produced.

In the invention, the temperature of the air stream is set to a melting point or higher of the thermoplastic resin composition.

In the invention, the heat of fusion of the thermoplastic resin composition is set to more than 5 mJ/mg and/or less than 94 mJ/mg.

In the invention, the temperature of the air stream is set to 260°C or less, optionally 250°C or less, and optionally 240°C or less.

In an embodiment, in the production method, the thermoplastic resin composition includes two or more different polyolefins having distinct heats of fusion.

In an embodiment, the thermoplastic resin composition includes a polyolefin compound including a first polyolefin having a heat of fusion of 94 mJ/mg or more and a second polyolefin having a heat of fusion of less than 94 mJ/mg.

In an embodiment, the second polyolefin includes at least one compound selected from a low crystalline polypropylene with an MFR of 400 g/10 min or more, a low crystalline polypropylene with an MFR of less than 400 g/10 min, and a polypropylene-based elastomer with an MFR of less than 400 g/10 min.

In an embodiment, the polyolefin compound includes at least one compound selected from a homopolymer of α-olefin or a copolymer of at least two different α-olefins.

In an embodiment, the α-olefin homopolymer includes at least one compound selected from a highly crystalline polyolefin or a low crystalline polyolefin.

In an embodiment, the copolymer of at least two different α-olefins includes at least one compound selected from a low crystalline olefin-based elastomer or an amorphous olefin-based elastomer.

In an embodiment, the first polyolefin includes a highly crystalline polyolefin, the highly crystalline polyolefin having a melt flow rate of 100 g/10 min or more and/or 2000 g/10 min or less.

In an embodiment, the first polyolefin includes a highly crystalline polyolefin, the highly crystalline polyolefin having a melt flow rate of 300 g/10 min or more and/or 1800 g/10 min or less.

In an embodiment, content of the second polyolefin with respect to the total amount of the first polyolefin and the second polyolefin is

50 mass% or higher and/or 70 mass% or lower when the second polyolefin is a low crystalline polypropylene with an MFR of 400 g/10 min or more, and

10 mass% or higher and/or 15 mass% or lower when the second polyolefin is a low crystalline polypropylene with an MFR of less than 400 g/10 min or a polypropylene-based elastomer with an MFR of less than 400 g/10 min.

In an embodiment, the production method further includes a deposition step of depositing the fiber obtained in the spinning step on a collection surface.

In an embodiment, in the production method, the distance between the nozzle and the collection surface is set to 400 mm or shorter, optionally 300 mm or shorter, and optionally 150 mm or shorter, and/or 50 mm or longer.

In an embodiment, the production method includes a heating step of heating the fiber obtained in the spinning step, before the fiber obtained in the spinning step is deposited on the collection surface.

In addition, the invention provides a meltblown non-woven fabric produced by the above production method.

Brief Description of Drawings

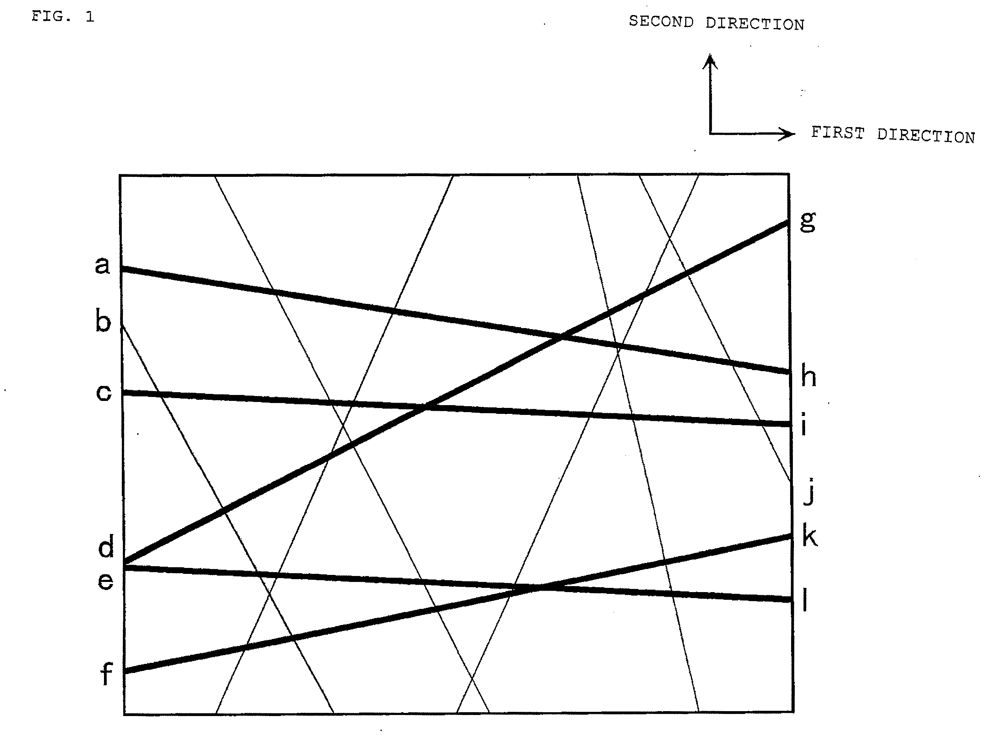

Fig. 1 is a schematic diagram illustrating an example of SEM image of a meltblown non-woven fabric according to the invention.

Description of Embodiments

Generally speaking, the non-woven fabric transfer direction during production of a meltblown non-woven fabric is referred to as a machine direction (MD direction) and a direction orthogonal to the MD direction is referred to as a cross direction (CD direction).

The present inventors have found that the strength of meltblown non-woven fabric in the MD direction is sufficient, but the strength in the CD direction is low. This may cause a marked decrease in water resistance of the meltblown non-woven fabric because an inter-fiber distance is likely to extend and a gap thus occurs when, for instance, a tensile load is applied in the CD direction and deformation thus occurs. Generally speaking, when a meltblown non-woven fabric is used at a site where deformation may occur due to a load application, a high-strength spunbonded non-woven fabric and/or an air-through non-woven fabric are layered and used so as to cover the meltblown non-woven fabric.

The invention relates to a meltblown non-woven fabric in which a decrease in water pressure resistance due to deformation is suppressed.

The meltblown non-woven fabric according to the invention can suppress the decrease in water pressure resistance due to deformation.

The meltblown non-woven fabric of the invention includes fibers with an average fiber diameter of 4 um or less. As the average fiber diameter becomes smaller, the water pressure resistance in a region with a low filling factor increases. The average fiber diameter is preferably 3.6 um or less, more preferably 3.2 um or less, still more preferably 3 um or less, still more preferably 2.5 um or less, and particularly preferably 2 um or less. In addition, the average fiber diameter is preferably 0.1 um or larger, more preferably 0.2 um or larger, and still more preferably 0.3 um or larger. Specifically, the average fiber diameter is preferably from 0.1 um to 4 um, more preferably from 0.2 um to 3.6 um, still more preferably from 0.3 um to 3.2 um, still more preferably from 0.3 um to 3 um, still more preferably from 0.3 um to 2.5 um, and particularly preferably from 0.3 um to 2 um.

It is considered that when the average fiber diameter is this lower limit or more, the strength is excellent and when the average fiber diameter is the upper limit or less, the meltblown non-woven fabric excels in water pressure resistance. The average fiber diameter of the meltblown non-woven fabric according to the invention can be calculated as follows. First, a scanning electron microscope is used to capture an SEM image on a visual field in which 20 to 60 fibers are seen. Fiber diameters with respect to all the fibers in the visual field are each measured once and averaged. The average is rounded off to the 10 nm place to give an average fiber diameter. The average fiber diameter is represented in $\mu$m.

The meltblown non-woven fabric according to the invention has: a first direction that is along a plane of the meltblown non-woven fabric and has the highest rate of straight-running fibers; and a second direction orthogonal to the first direction. The rate of straight-running fibers in the first direction and the rate of straight-running fibers in the second direction are each preferably 35% or higher, more preferably 38% or higher, still more preferably 40% or higher and preferably 90% or lower, more preferably 85% or lower, and still more preferably 80% or lower. Specifically, the rates are preferably from 35% to 90%, more preferably from 38% to 85%, and still more preferably from 40% to 80%. It is preferable that the first direction is the MD direction during the production. It is preferable that the second direction is the CD direction during the production. When the rate of straight-running fibers in the first direction and the rate of straight-running fibers in the second direction are each this lower limit or more, an inter-fiber gap is unlikely to be formed when deformation occurs in any of the first direction or the second direction. This suppresses a decrease in the water pressure resistance.

Regarding a meltblown non-woven fabric according to the invention, the first direction is a direction that is along a plane of the meltblown non-woven fabric and has the highest rate of straight-running fibers; and the second direction is a direction orthogonal to the first direction. The ratio (A/B) of the rate (A) of straight-running fibers in the first direction to the rate (B) of straight-running fibers in the second direction is 1 or higher and 2.5 or lower, more preferably 2.1 or lower, and still more preferably 1.9 or lower. Specifically, the ratio (A/B) is from 1 to 2.5, more preferably from 1 to 2.1, and still more preferably from 1 to 1.9. When the ratio (A/B) is this upper limit or less, an inter-fiber gap is unlikely to be formed even when deformation occurs in any of the first direction or the second direction. This suppresses a decrease in the water pressure resistance.

The meltblown non-woven fabric according to the invention has a water pressure resistance of preferably 100 mm $H_2O$ or higher and preferably 10000 mm $H_2O$ or lower. When the meltblown non-woven fabric is deformed in the second direction orthogonal to the first direction that is along a plane of the meltblown non-woven fabric and has the highest rate of straight-running fibers, the water pressure resistance retention rate is preferably 85% or higher, more preferably 88% or higher, and still more preferably 90% or higher. In addition, realistically speaking, the water pressure resistance retention rate is 100% or less. If the water pressure resistance retention rate of the meltblown non-woven fabric according to the invention when deformed in the second direction is this lower limit or higher, an inter-fiber gap is hardly formed even upon deformation in any of the first direction or the second direction. This can suppress a decrease in the water pressure resistance even if deformation occurs upon usage without layering a spunbonded non-woven fabric or an air-through non-woven fabric.

The following steps (a) to (g) are used to determine the first direction having the highest rate of straight-running fibers of the meltblown non-woven fabric according to the invention, the second direction orthogonal to the first direction as well as the rate (A) of straight-running fibers in the first direction and the rate (B) of straight-running fibers in the second direction. Note that the "first direction having the highest rate of straight-running fibers" of the meltblown non-woven fabric according to the invention means a direction determined through the following steps (a) to (g) and is sometimes different from an actual direction having the highest rate of straight-running fibers.

(a) Using a desktop scanning electron microscopy (JCM-6000 Plus, manufactured by JEOL Ltd.) to capture an SEM image of the meltblown non-woven fabric, an observation position of which is a center portion, at an observation magnification = 3000/an average fiber diameter ($\mu$m) and obtain the SEM image such that a long-side direction of the SEM image is set to 0 degrees, provided that when a non-woven fabric to be measured has a substantially rectangular shape, a longitudinal direction of the non-woven fabric is set to 0 degrees and the long-side direction of the SEM image is parallel to the 0 degrees direction;

(b) capturing an SEM image when a visual field is rotated $\theta$ degrees and obtaining X pieces of the SEM images at from 0 degrees to (180 - $\theta$) degrees when the visual field is likewise rotated by every $\theta$ degrees, where the number X of pieces = (180 degrees/$\theta$ degrees)-1;

(c) performing manipulations in steps (a) and (b) with respect to 7 observation positions other than the observation position in step (a) to obtain the SEM images at X pieces $\times$ 8 positions, provided that the 8 observation positions are located within an area of 40 mm $\times$ 20 mm at the center portion of the meltblown non-woven fabric and are apart from one another by 10 mm;

(d) calculating, using the following Equation (1), a rate of straight-running fibers at each angle from the 0 degrees to the (180 - $\theta$) degrees with respect to the respective SEM images obtained through steps (a) to (c), and rounding the rate off to the closest whole number, and averaging the rates at the 8 positions to yield a straight-running rate at each angle;

(e) setting an angle with the largest straight-running rate among the straight-running rates calculated in step (d) for each angle to a first direction and setting the straight-running rate with the largest value to a rate (A) of straight-running fibers in the first direction;

(f) setting a direction orthogonal to the first direction to a second direction; and

(g) obtaining SEM images, a long-side direction of which is parallel to the second direction, with respect to the 8 observation positions, calculating respective rates (B) of straight-running fibers in the second direction by using the following expression (1), and averaging and rounding the rates at the 8 positions off to the closest whole number:

[Expression 2]

$$\text{Straight-running rate } (\%) = (N(0) \times 2 / (N(1) + N(2))) \times 100 \ ... \ (1)$$

where respective N(0), N(1), and N(2) represent the following, wherein

N(0) is the number of fibers that continuously extend from one end to another end of each SEM image in the long-side direction;

N(1) is the number of fibers that intersect the one end in the long-side direction; and

N(2) is the number of fibers that intersect the other end in the long-side direction.

The fiber reaching the one end of each SEM image in the long-side direction is counted as a "fiber that intersects the one end in the longitudinal direction". The same applies to the other end.

Here, a procedure for calculating a rate of straight-running fibers in step (d) is described in detail with reference to Fig. 1. Fig. 1 is a schematic diagram illustrating an example of SEM image, the long-side direction of which is parallel to the first direction. Note that in Fig. 1, the fibers are depicted as straight lines because the illustration is simplified. The shape of actual fibers, however, is not limited to a linear shape. In addition, the number of fibers seen in each SEM image may be different from the actual number.

In the case of the SEM image shown in Fig. 1, the number of fibers that intersect a left end in the long-side direction is 6 involving intersection points a to f and the number of fibers that intersect a right end in the long-side direction is 6 involving intersection points g to l. The total number of fibers that continuously extend from the left end to the right end in the long-side direction is 5 involving straight line ah, straight line ci, straight line dg, straight line el, and straight line fk. Thus, the straight-running rate in the first direction (A)(%) = [5 × 2/(6 + 6)] × 100 = 83%.

In step (b), the angle θ degrees when the visual field is altered may be any value and preferably 15 degrees or less, more preferably 10 degrees or less, and still more preferably 5 degrees or less. Meanwhile, when the above-described measurement is conducted multiple times, the conditions such as the observation positions and/or the angle θ may be different and as a result of which each determined "direction having the highest straight-running rate" may be different. In this case, a direction having the highest straight-running rate among the multiple measurement results may be adopted as the first direction.

When the rate of straight-running fibers of the meltblown non-woven fabric according to the invention is 35% or higher in each of the first direction having the highest rate of straight-running fibers and the second direction orthogonal thereto, an inter-fiber gap hardly occurs upon deformation. This can suppress a decrease in the water pressure resistance. Further, even if the meltblown non-woven fabric according to the invention is used without layering a spunbonded non-woven fabric or an air-through non-woven fabric and is then deformed, it is possible to suppress a decrease in the water pressure resistance.

The decrease in the water pressure resistance due to deformation of the meltblown non-woven fabric according to the invention can be evaluated using a ratio (water pressure resistance retention rate) of post-deformation water pressure resistance to pre-deformation water pressure resistance. The water pressure resistance and the water pressure resistance retention rate may be measured by the below-described protocol. The meltblown non-woven fabric may be integrated with a backing, such as a spunbonded non-woven fabric or an air-through non-woven fabric, by thermal embossing. The integrated meltblown non-woven fabric is subjected to drawing in an integrated state and the water pressure resistance is then measured. In the case where the meltblown non-woven fabric is integrated with a resin film by thermal embossing, only a film on a non-embossed region is removed and the corresponding fabric is to be measured.

The meltblown non-woven fabric according to the invention may be obtained by adjusting a heat of fusion of thermoplastic resin composition as a component of fiber, by adjusting the temperature of air stream during the spinning step, or by heating the fiber after the spinning step and before the deposition step. The temperature of air stream during the spinning step and the heating of the fiber will be described later. Note that the thermoplastic resin composition includes at least one thermoplastic resin and is a mixture optionally including another component, if appropriate.

From the viewpoint of spinnability, the heat of fusion of thermoplastic resin composition is preferably larger than 5 mJ/mg, more preferably 10 mJ/mg or higher, and particularly preferably 20 mJ/mg or higher. In addition, from the viewpoint of producing soft fiber and the viewpoint of making fiber thin in a low-temperature hot-wind-temperature region, the heat of fusion is preferably less than 94 mJ/mg, more preferably 90 mJ/mg or less, still more preferably 80 mJ/mg or less, still more preferably 75 mJ/mg or less, still more preferably 45 mJ/mg or less, and particularly preferably 35 mJ/mg or less.

Specifically, the heat of fusion is preferably more than 5 mJ/mg but less than 94 mJ/mg, more preferably from 10 mJ/mg to 90 mJ/mg, still more preferably from 20 mJ/mg to 80 mJ/mg, still more preferably from 20 mJ/mg to 75 mJ/mg, still more preferably from 20 mJ/mg to 45 mJ/mg, and particularly preferably from 20 mJ/mg to 35 mJ/mg.

The heat of fusion is an indicator for indicating flexibility, that is, how much crystalline region exists in a thermoplastic resin composition. The heat of fusion of thermoplastic resin composition can be set to be within a desired range by adjusting a thermoplastic resin used and its content. The heat of fusion of thermoplastic resin composition can be calculated by the below-described protocol after 1 mg of measurement piece is sampled from a center portion of the meltblown non-woven fabric.

When the heat of fusion is less than the above upper limit, how much amorphous region occupies the fiber is large. As a result, the fiber is softened and the orientation occurring during the spinning step is suppressed. The fiber orientation will be described later. When the heat of fusion is larger than the above lower limit, the fiber is softened and the resulting orientation is unlikely to occur. Meanwhile, when the heat of fusion is less than the above upper limit, crystalline regions present in the fiber are sufficiently many, fusion between the fibers is suppressed, and an inter-fiber distance is narrowed, so that the water pressure resistance can be improved.

The thermoplastic resin composition may singly include a thermoplastic resin having a heat of fusion within a desired range. In addition, to control a heat of fusion within a desired range, two or more different thermoplastic resins with distinct heats of fusion may be mixed. When a plurality of thermoplastic resins are used, a thermoplastic resin with a heat of fusion of the upper limit of the desired range or more and a thermoplastic resin with a heat of fusion of the lower limit of the desired range or less may be blended to have a heat of fusion within the desired range. Alternatively, a plurality of thermoplastic resins, each having a heat of fusion within the desired range, may be blended.

Examples of the thermoplastic resin that can be used include polyolefin, polyester, polyetheretherketone, polyphenylene sulfide, or polyamide. Among them, polyolefin or polyester is preferable, and polyolefin is particularly preferable. As long as the heat of fusion is within the desired range, one kind of these thermoplastic resins may be used singly, or two or more kinds may be used in combination.

It is preferable that the thermoplastic resin composition contains a polyolefin in an amount of 70 mass% or higher. The polyolefin more preferably occupies 80 mass% or more and still more preferably occupies 90 mass% or more of the thermoplastic resin composition.

As the polyolefin, it is possible to use a homopolymer of $\alpha$-olefin or a copolymer of at least two different $\alpha$-olefins. They may be used singly, or two or more kinds thereof may be used in combination. As the polyolefin, it is possible to use, for instance, a copolymer of $\alpha$-olefin and any of an unsaturated carboxylic acid, such as acrylic acid, methacrylic acid, or maleic acid, an ester of the unsaturated carboxylic acid, or an acid anhydride.

The $\alpha$-olefin preferably contains from 2 to 20 carbon atoms and more preferably contains from 2 to 10 carbon atoms. As the $\alpha$-olefin, propylene, ethylene, 1-butene, 1-hexene, 1-octene, 4-methyl-1-pentene, or the like is preferable; propylene or ethylene is more preferable; and propylene is most preferable.

As the $\alpha$-olefin homopolymer, it is possible to use a highly crystalline polyolefin or a low crystalline polyolefin. The highly crystalline polyolefin is a polyolefin having a highly stereoregular $\alpha$-olefin. Specific examples of the highly crystalline polyolefin include: a highly crystalline polypropylene such as an isotactic polypropylene or a syndiotactic polypropylene; and a highly crystalline polyethylene such as a high-density polyethylene or a medium-density polyethylene.

Each highly crystalline polypropylene commonly used for meltblown non-woven fabrics has a heat of fusion of more than 94 mJ/mg and is a hard resin with large crystalline regions. Because a shear flow occurs on a spinning line during the spinning step, a rigid fiber with large crystalline regions is readily oriented in the air stream direction. The air stream is blown onto a collection surface that moves in the MD direction. Accordingly, the fiber should be deposited on the collection surface while the orientation direction is the same as the MD direction. Thus, the heat of fusion of thermoplastic resin composition as a component of the fiver is set to less than 94 mJ/mg. This makes it unlikely to orient the fiber in the shear direction because there are many soft amorphous regions in the fiber. As a result, a non-woven fabric deposited on the collection surface should have a smaller fiber orientation in the MD direction. Consequently, the straight-running rate in the CD direction should be high.

The heat of fusion of the highly crystalline polyolefin is preferably larger than 94 mJ/mg, more preferably 96 mJ/mg or higher, and still more preferably 98 mJ/mg or higher and preferably less than 120 mJ/mg, more preferably 115 mJ/mg or lower, and still more preferably 110 mJ/mg or lower. Specifically, the heat of fusion is larger than 94 mJ/mg but less than 120 mJ/mg, more preferably from 96 mJ/mg to 115 mJ/mg, and still more preferably from 98 mJ/mg to 110 mJ/mg.

The melt flow rate (MFR) (at 230°C) of the highly crystalline polyolefin is preferably 100 g/10 min or higher and more preferably 300 g/10 min or higher and preferably 2000 g/10 min or lower and more preferably 1800 g/10 min or lower. Specifically, the melt flow rate is preferably from 100 g/10 min to 2000 g/10 min and more preferably from 300 g/10 min to 1800 g/10 min. The MFR can be measured, based on JIS K7210, at a load of 2.16 kg and a temperature of 230°C. When the MFR is this upper limit or lower, the resin fluidity during the spinning step is not too high. This prevents thread breakage, thereby easily realizing fine fibers. Also, when the MFR is this lower limit or higher, the resin is flowable. This makes it possible to sufficiently draw the fiber during the spinning step, thereby capable of making the fiber diameter smaller.

The highly crystalline polyolefin has a weight-average molecular weight (Mw) of preferably 5000 or higher, more preferably 10000 or higher, and still more preferably 15000 or higher and preferably 500000 or lower, more preferably 200000 or lower, and still more preferably 150000 or lower. Specifically, the Mw is preferably from 5000 to 500000, more preferably from 10000 to 200000, and still more preferably from 15000 to 150000. When the weight-average molecular weight is this lower limit or higher, the polymer chains are strongly entangled during the spinning step. This prevents thread breakage, thereby capable of realizing fine fibers. Also, when the weight-average molecular weight is this upper limit or lower, the polymer chains are not entangled too strongly. This makes it possible to sufficiently draw the fiber during the spinning step, thereby capable of making the fiber diameter smaller.

The highly crystalline polyolefin has a molecular weight distribution (weight-average molecular weight (Mw)/number-average molecular weight (Mn)) of preferably 1.1 or higher, more preferably 1.5 or higher, and still more preferably 2 or higher and preferably 5 or lower, more preferably 4 or lower, and still more preferably 3.5 or lower. Specifically, the molecular weight distribution is preferably from 1.1 to 5, more preferably from 1.5 to 4, and still more preferably from 2 to 3.5.

The low crystalline polyolefin is a polyolefin containing a low stereoregular α-olefin. Specific examples of the low crystalline polyolefin include a low crystalline polypropylene such as an atactic polypropylene or a low stereoregular polypropylene; or a low crystalline polyethylene such as a low-density polyethylene or a linear low-density polyethylene. The low stereoregular polypropylene can be obtained by polymerizing propylene by using a publicly known meta-cellon catalyst.

The heat of fusion of the low crystalline polyolefin is preferably larger than 0 mJ/mg, more preferably 3 mJ/mg or higher, and still more preferably 5 mJ/mg or higher and preferably less than 94 mJ/mg, more preferably 85 mJ/mg or lower, and still more preferably 70 mJ/mg or lower. Specifically, the heat of fusion is larger than 0 mJ/mg but less than 94 mJ/mg, more preferably from 3 mJ/mg to 85 mJ/mg, and still more preferably from 5 mJ/mg to 70 mJ/mg.

The MFR (at 230°C) of the low crystalline polyolefin is preferably 100 g/10 min or higher, more preferably 1000 g/10 min or higher, and still more preferably 1800 g/10 min and preferably 2500 g/10 min or lower, more preferably 2300 g/10 min or lower, and still more preferably 2100 g/10 min or lower. Specifically, the MFR is preferably from 100 g/10 min to 2500 g/10 min, more preferably from 1000 g/10 min to 2300 g/10 min, and still more preferably from 1800 g/10 min to 2100 g/10 min. The MFR can be measured, based on JIS K7210, at a load of 2.16 kg and a temperature of 230°C.

The low crystalline polyolefin has a weight-average molecular weight (Mw) of preferably 5000 or higher, more preferably 20000 or higher, and still more preferably 30000 or higher and preferably 150000 or lower, more preferably 70000 or lower, and still more preferably 50000 or lower. Specifically, the Mw is preferably from 5000 to 150000, more preferably from 20000 to 70000, and still more preferably from 30000 to 50000.

The copolymer of two or more different α-olefins is a low crystalline or amorphous olefin-based elastomer. As the α-olefin copolymer, it is possible to use a random copolymer, a block copolymer, a graft copolymer, or an alternating copolymer. In the case of the block copolymer, it is preferable that the α-olefins are linked using an atactic structure. Hereinafter, the copolymer of two or more different α-olefins is referred to as an olefin-based elastomer.

The olefin-based elastomer has a heat of fusion of larger than 0 mJ/mg but less than 94 mJ/mg, preferably 3 mJ/mg or larger, more preferably 5 mJ/mg or larger and preferably 90 mJ/mg or less and more preferably 85 mJ/mg or less.

The olefin-based elastomer may optionally include, in addition to the α-olefins, at least one kind selected from the group consisting of polyene compound units such as butadiene, isoprene, ethylidene norbornane, and dicyclopentadiene units, cyclic olefin units, and vinyl aromatic compound units as a monomer(s).

Specific examples of the olefin-based elastomer include a propylene-ethylene copolymer, a propylene-ethylene-1-butene copolymer, a propylene-1-butene copolymer, a propylene-ethylene-cyclic olefin copolymer, a propylene-ethylene-butadiene copolymer, or a propylene-1-butene-styrene copolymer. Among these copolymers, a propylene-ethylene copolymer or a propylene-ethylene-1-butene copolymer is most preferable. One kind of them may be used singly or two or more kinds may be used in combination.

The MFR (at 230°C) of the olefin-based elastomer is preferably 10 g/10 min or higher and more preferably 300 g/10 min or higher and preferably 2000 g/10 min or lower and more preferably 1800 g/10 min or lower. The MFR can be measured, based on JIS K7210, at a load of 2.16 kg and a temperature of 230°C.

The olefin-based elastomer has a molecular weight distribution (weight-average molecular weight (Mw)/number-average molecular weight (Mn)) of preferably 1.1 or higher, more preferably 1.3 or higher, and still more preferably 1.5 or higher and preferably 5 or lower, more preferably 4 or lower, and still more preferably 3.5 or lower.

The olefin-based elastomer may be produced using a polymerization catalyst such as a publicly known Ziegler-Natta catalyst or a single-site catalyst (e.g. a metallocene catalyst).

It is preferable that the thermoplastic resin composition contains a highly crystalline polyolefin and a low crystalline polyolefin or a polyolefin-based elastomer.

It is preferable that the thermoplastic resin composition contains a mixture of a first polyolefin with a heat of fusion of 94 mJ/mg or higher and a second polyolefin with a heat of fusion of less than 94 mJ/mg.

In meltblown spinning, fiber is formed by stretching a molten resin. When threading and/or thread breakage prevention are taken into consideration, it is particularly preferable that the thermoplastic resin composition contains a mixture of a highly crystalline polypropylene that is commonly used in meltblowing and has a heat of fusion of 94 mJ/mg or higher and a low crystalline polypropylene or a polypropylene-based elastomer that has a heat of fusion of less than 94 mJ/mg.

Preferable specific examples of the second polyolefin include a highly flowable low crystalline polypropylene with a MFR of 400 g/10 min or higher, a less flowable low crystalline polypropylene with an MFR of less than 400 g/10 min, or a less flowable polypropylene-based elastomer with an MFR of less than 400 g/10 min.

The content of the highly flowable low crystalline polypropylene with an MFR of 400 g/10 min or higher is preferably 90 mass% or less, more preferably 80 mass% or less, and still more preferably 70 mass% or less with respect to the total of the thermoplastic resin composition. The content of the highly flowable low crystalline polypropylene is preferably 3 mass% or higher, more preferably 10 mass% or higher, and still more preferably 20 mass% or higher, and still more preferably 50 mass% or higher. Specifically, the content is preferably from 3 mass% to 90 mass%, more preferably from 10 mass% to 80 mass%, still more preferably from 20 mass% to 70 mass%, and still more preferably from 50 mass% to 70 mass%.

When the content of the highly flowable low crystalline polypropylene is this upper limit or less, amorphous regions in the fiber are not too many. This can prevent fusion between the fibers and make an inter-fiber distance shorter, thereby capable of improving the water pressure resistance. Also, when the content of the highly flowable low crystalline polypropylene is this lower limit or higher, amorphous regions in the fiber are adequately present and make the fiber soft. As a result, the orientation during the spinning step is unlikely to occur.

The content of the low crystalline polypropylene with an MFR of less than 400 g/10 min or the polypropylene-based elastomer with an MFR of less than 400 g/10 min is preferably 30 mass% or less, more preferably less than 30 mass%, still more preferably 20 mass% or less, and still more preferably 15 mass% or less with respect to the total of the thermoplastic resin composition. The content of the polypropylene-based elastomer is preferably 3 mass% or higher, more preferably 5 mass% or higher, still more preferably 10 mass% or higher, and particularly preferably 20 mass% or higher. Specifically the content is preferably from 3 mass% to 30 mass%, more preferably from 3 mass% to less than 30 mass%, still more preferably from 5 mass% to 20 mass%, still more preferably from 10 mass% to 20 mass%, particularly preferably from 20 mass% to 15 mass%, and most preferably from 10 mass% to 15 mass%.

Generally speaking, the low crystalline polypropylene with an MFR of less than 400 g/10 min and the polypropylene-based elastomer with an MFR of less than 400 g/10 min have lower fluidity than polypropylene resins for meltblowing. When the content is this upper limit or lower, the whole resin fluidity is increased. This makes it possible to sufficiently draw the fiber during the spinning step, thereby making the fiber diameter smaller. In order to enlarge amorphous regions in the fiber and realize fine fibers during the spinning step, it is preferable that the content of the low crystalline polypropylene with an MFR of less than 400 g/10 min or the polypropylene-based elastomer with an MFR of less than 400 g/10 min is within the above ranges.

Specific examples of the first polyolefin include a highly crystalline polypropylene. The mass-based blending ratio between the first polypropylene and the second polypropylene (the first polypropylene/the second polypropylene) is selected depending on their kinds.

For instance, when Moplen (registered trademark) HP461Y (manufactured by Lyondellbasell, Inc.) is used as the first polypropylene (highly crystalline polypropylene) and L-MODU (registered trademark) S400 (manufactured by Idemitsu Kosan Co., Ltd.) with an MFR of 2600 g/10 min is used as the second polypropylene (highly flowable low crystalline polypropylene), the blending ratio is preferably larger than 5/95, more preferably 10/90 or larger, still more preferably 20/80 or larger, still more preferably 30/70 or larger and preferably 97/3 or less, more preferably 90/10 or less, still more preferably 80/20 or less, and still more preferably 50/50 or less. Specifically, the blending ratio is preferably from larger than 5/95 to 97/3, more preferably from 10/90 to 90/10, still more preferably from 20/80 to 80/20, and still more preferably from 30/70 to 50/50.

Specific examples of the less flowable low crystalline polypropylene that can be used as the second polypropylene include L-MODU (registered trademark) S600 with an MFR of 350 g/10 min or L-MODU (registered trademark) S901 with an MFR of 50 g/10 min (the both manufactured by Idemitsu Kosan Co., Ltd.). Examples of the polypropylene-based elastomer that can be used as the second propylene include TAFTHREN (registered trademark) H5002 with an MFR of 10 g/10 min (manufactured by Sumitomo Chemical Industry Company Limited).

When such a second polypropylene is used together with Moplen (registered trademark) HP461Y (manufactured by Lyondellbasell, Inc.) as the first polypropylene, the blending ratio (the first polypropylene/the second polypropylene) is preferably 70/30 or larger, more preferably 75/25 or larger, still more preferably 80/20 or larger, and still more preferably 85/15 or larger and preferably 97/3 or less, more preferably 95/5 or less, and still more preferably 90/10 or less. Specifically, the blending ratio is preferably from 70/30 to 95/5, more preferably from 75/25 to 95/5, still more preferably from 80/20 to 90/10, and still more preferably from 85/15 to 90/10.

As the polyester, for instance, polyethylene terephthalate, polybutylene terephthalate, or polytrimethylene terephthalate may be used. Among them, polyethylene terephthalate or polybutylene terephthalate is preferable. When plural different polyesters are mixed and used, the content of any of the polyesters is preferably 50 mass% or larger, more preferably 70 mass% or larger, and still more preferably 90 mass% or larger with respect to the whole polyesters.

As the polyamide, for instance, polyamide 3, polyamide 4, polyamide 6, polyamide 66, or polyamide 12 may be used.

The thermoplastic resin composition may contain an additive such as a nucleating agent, a dulling agent, a pigment, a dye, an antifungal, an antimicrobial, a flame retardant, a hydrophilic agent, a photostabilizer, an antioxidant, an aging inhibitor, synthetic oil, wax, a coloring inhibitor, and/or a viscosity modifier, to the extent without impairing the advantages of the invention.

The filling factor of the meltblown non-woven fabric according to the invention is preferably 3% or higher, more preferably 5% or higher, and still more preferably 6% or higher. As the filling factor becomes larger, the fibers are present more densely and the water pressure resistance of the meltblown non-woven fabric is thus increased. As the filling factor is increased, the meltblown non-woven fabric becomes more rigid. As the filling factor is lowered, the meltblown non-woven fabric becomes softer. A soft meltblown non-woven fabric is preferred when used as clothing. Accordingly, the filling factor is preferably 30% or less, more preferably 20% or less, and still more preferably 15% or less. The filling factor of the meltblown non-woven fabric according to the invention is preferably from 3% to 30%, more preferably from 5% to 20%, and still more preferably from 6% to 15%.

The filling factor can be made the above lower limit or higher by setting, to 400 mm or shorter, the distance from the nozzles to the collection surface of the below-described non-woven fabric production machine, thereby making the resulting fiber compact by using the blowing pressure of air stream. In addition, the meltblown non-woven fabric is compacted using, for instance, a calendar roll during production and the filling factor can then be adjusted to the above lower limit or higher.

In order to calculate the filling factor, firstly, the thickness of a meltblown non-woven fabric obtained by the below-described procedure is measured by using a laser displacement meter manufactured by OMRON Corporation while a load is imposed to the non-woven fabric such that a pressure of 4 kPa is applied thereto. The filling factor is determined using the below-described Expression (2) in accordance with the below-described protocol.

The formation index of the meltblown non-woven fabric according to the invention is preferably 300 or less, more preferably 280 or less, still more preferably 260 or less, still more preferably 250 or less, and particularly preferably 200 or less. The formation index can be made the above upper limit or less by setting, to 400 mm or less, the distance from the nozzles to the collection surface of the below-described non-woven fabric production machine, thereby preventing unevenness from occurring while the fibers are deposited.

The formation index when a non-woven fabric is produced by meltblowing is at least about 30 in practice. As the formation index becomes smaller, the fibers are present more uniformly. This causes the water pressure resistance of the meltblown non-woven fabric to become larger. The formation index is measured, using the below-described protocol, at random positions in the longitudinal direction, which positions are in a center portion in the widthwise direction of the meltblown non-woven fabric.

Note that the meltblown non-woven fabric, which has been integrated with a backing such as a spunbonded non-woven fabric or an air-through non-woven fabric by thermal embossing, may have a hole created when a sample is obtained by peeling. The formation index of the sample with an open hole is calculated by using the standard deviation and the average of absorbance obtained after the hole-derived absorbance E is subtracted.

From the viewpoint of setting the filling factor and the formation index to the above ranges, the basis weight of the meltblown non-woven fabric according to the invention is preferably 20 g/m² or less, more preferably 15 g/m² or less, and still more preferably 10 g/m² or less. When the basis weight is this upper limit or less, the fiber has a small volume in response to the blowing pressure of air stream and is thus easily compacted. This causes the filling factor to be sufficient. Also, when the basis weight is this upper limit or less, the fibers are uniformly deposited during the deposition step and the formation index is thus adequate.

To cause the meltblown non-woven fabric to express water pressure resistance, the basis weight is preferably 1 g/m² or larger and more preferably 2 g/m² or larger. Specifically, the basis weight of the meltblown non-woven fabric according to the invention is preferably from 1 g/m² to 20 g/m², more preferably from 1 g/m² to 15 g/m², and still more preferably from 2 g/m² to 10 g/m² or less.

When the meltblown non-woven fabric is a single body, the basis weight may be determined from the weight per 0.05 m square area. When the meltblown non-woven fabric is a composite obtained by bonding it to a backing such as a resin film or paper, a spunbonded non-woven fabric, or an air-through non-woven fabric by using a hotmelt, etc., the fabric is first treated with a cold spray or heated with, for instance, a dryer to lower the bonding strength of the hotmelt. Then, the meltblown non-woven fabric is peeled off from the backing. The hotmelt attached to the meltblown non-woven fabric is dissolved by soaking the meltblown non-woven fabric in a hotmelt-miscible organic solvent, such as toluene, in large excess for 24 h. The meltblown non-woven fabric is collected from the organic solvent and is then dried. After that, the basis weight of the meltblown non-woven fabric is measured by the above procedure.

Note that a procedure for collecting this meltblown non-woven fabric from the composite is applicable to the other measurements used herein. When the meltblown non-woven fabric is integrated with a backing such as a resin film, a spunbonded non-woven fabric, or an air-through non-woven fabric by thermal embossing, the meltblown non-woven fabric is first peeled off so as to remove embossed portions. Next, the area of the meltblown non-woven fabric in a state in which holes are created at the embossed portion sites is calculated by image processing such as binarization. Then, the weight at that time may be used to determine the basis weight.

The meltblown non-woven fabric that has a 0.05 m-square area and is subject to the basis weight measurement is preferably obtained from a continuous meltblown non-woven fabric. If the area of a piece of the meltblown non-woven fabric that can be obtained from a product is small, the sum of areas of a plurality of meltblown non-woven fabrics obtained from the product may be used.

When the meltblown non-woven fabric is integrated with a backing such as a resin film, a spunbonded non-woven fabric, or an air-through non-woven fabric by thermal embossing, the meltblown non-woven fabric is peeled off, if appropriate, such that embossed portions are not included, and is to be measured except for the below-described water pressure resistance measurement and post-deformation water pressure resistance measurement.

As described above, the meltblown non-woven fabric according to the invention has an average fiber diameter of 4 um or less and the rate of straight-running fibers in each of a first direction that is along a plane thereof and has the highest rate of straight-running fibers and a second direction orthogonal to the first direction is 35% or higher. Consequently, the water pressure resistance is excellent, an inter-fiber gap is unlikely to occur upon deformation, and a decrease in the water pressure resistance can be suppressed.

The water pressure resistance of the meltblown non-woven fabric may be measured by obtaining a meltblown non-woven fabric by using substantially the same way as above. In this regard, however, when the meltblown non-woven fabric is integrated with a backing such as a spunbonded non-woven fabric or an air-through non-woven fabric by thermal embossing, the water pressure resistance is measured while the form is as it is and the value is defined as the water pressure resistance of the meltblown non-woven fabric. In the case of such a layered non-woven fabric, the finely meshed meltblown non-woven fabric is a layer that determines the water pressure resistance. Accordingly, the water pressure resistance of the meltblown non-woven fabric is considered to be the water pressure resistance of the whole layered non-woven fabric.

Applications of the meltblown non-woven fabric according to the invention are not particularly limited and the meltblown non-woven fabric can be used for various applications taking advantage of its characteristics. The meltblown non-woven fabric according to the invention may be used as a single layer or may be layered. A plurality of the meltblown non-woven fabrics according to the invention may be layered or the meltblown non-woven fabric and another publicly known non-woven fabric such as a spunbonded non-woven fabric or an air-through non-woven fabric are layered together. Further, the meltblown non-woven fabric according to the invention may be subjected to embossing, if necessary.

Specifically, a spunbonded layer and a meltblown layer may each be produced by spinning and they are then layered and integrated by thermal embossing. Alternatively, after a spunbonded non-woven fabric is produced by spinning, a separately prepared meltblown non-woven fabric may be layered and embossed. Further, a meltblown non-woven fabric and a spunbonded non-woven fabric may be separately produced and then integrated by thermal embossing. In either case, the meltblown non-woven fabric may be peeled off, if appropriate, such that embossed portions are not included in the same manner as above, and the basis weight may then be measured.

The meltblown non-woven fabric according to the invention is applicable to structural members of absorption article such as a disposable diaper, a sanitary napkin, or an incontinence pad, and is suitable as a leakage prevention sheet that requires water resistance, in particular, because a decrease in the water pressure resistance due to deformation is suppressed. Such an absorption article may be produced by layering a leakage prevention sheet made of the meltblown non-woven fabric according to the invention, an absorbent, and a surface sheet. In addition, the meltblown non-woven fabric according to the invention may be used for a hygiene mask, a liquid filter, an air filter, a battery separator, a glove, or the like.

Next, a method of producing a meltblown non-woven fabric according to the invention will be described. The meltblown non-woven fabric according to the invention may be produced by meltblowing using a publicly known non-woven fabric production machine that has been conventionally used during manufacture of meltblown non-woven fabrics.

The non-woven fabric production machine, for instance, is provided with an extruder equipped with a barrel having a built-in screw and a raw material inlet; a die connected directly or via a gear pump, etc., to the extruder; and a collection surface for depositing a fiber-shaped molten material. In the die, multiple nozzles for discharging a molten material are arranged in series. Blowing ports are provided on both sides of each nozzle, a high-temperature and high-pressure air stream (hot wind) is jetted from the blowing ports, and the molten material discharged from each nozzle is drawn to produce a fiber. The multiple nozzles are preferably arranged in series with a constant interval. It is preferable that the diameter of each nozzle is several hundred um. The high-temperature and high-pressure air stream is preferably an air stream, but may be another gas stream. As the collection surface, a publicly known surface such as a net conveyer or a collection screen may be used.

The method of producing a meltblown non-woven fabric according to the invention is a method of producing a meltblown non-woven fabric having an average fiber diameter of 4 um or less, the method including a spinning step of supplying a thermoplastic resin composition, for instance, in a pellet form from a raw material inlet to an extruder, heating and melting it in the extruder, then feeding the resulting molten material to a die and discharging the material from each nozzle, and drawing the discharged molten material by using a high-temperature and high-pressure air stream (hot wind) to produce a fiber. The fiber-shaped molten material is deposited on the collection surface during the deposition step and the fibers are fused to one another to produce a meltblown non-woven fabric.

It is possible to use any of the above-described thermoplastic resin compositions as the thermoplastic resin composition used in the method of producing a meltblown non-woven fabric according to the invention. The thermoplastic resin composition may be prepared by directly injecting a thermoplastic resin and an optionally blended component, instead of the pellet, to the extruder.

In the method of producing a meltblown non-woven fabric according to the invention, the temperature (hot wind temperature) of air stream during the spinning step is 260°C or lower, preferably 250°C or lower, and more preferably 240°C or lower. When the air stream temperature is this upper limit or lower, the time during which the fiber is tacky is shortened. This can cause the fibers to be unlikely entangled, suppress air resistance to the fiber, less orient the fiber in the MD direction during the spinning step, and increase the rate of straight-running fibers in the CD direction. Also, the lower limit of the air stream temperature should be the melting point or higher of the thermoplastic resin composition. The melting point is measured by differential scanning calorimetry (DSC). The temperature is raised from 30°C at a rate of 20°C/min to yield a DSC curve. Then, the melting point is set to the temperature of an endothermic peak at the highest temperature seen between 30 and 240°C.

It is preferable that the thermoplastic resin composition contains a highly crystalline polypropylene. The hot wind temperature is preferably 160°C, which is the melting point of the highly crystalline polypropylene, or higher, more preferably 180°C or higher, and still more preferably 200°C or higher. Specifically, the hot wind temperature is preferably from 160°C to 260°C, more preferably from 180°C to 250°C, and still more preferably from 200°C to 240°C.

From the viewpoint of reliably setting, to less than 4 um, the average fiber diameter of the meltblown non-woven fabric according to the invention, the flow rate of air stream during the spinning step is preferably 500 $Nm^3$/hr or higher and more preferably 700 $Nm^3$/hr or higher per air stream blowing width of 1 m. In addition, thread breakage during a fiber-drawing process should be prevented and as a result of which the fiber diameter should be made smaller. For this purpose, the air stream flow rate is preferably 1700 $Nm^3$/hr or lower and more preferably 1300 $Nm^3$/hr or lower per air stream blowing width of 1 m.

In the method of producing a meltblown non-woven fabric according to the invention, the distance from the nozzles to the collection surface of the non-woven fabric production machine is preferably 400 mm or shorter, more preferably 300 mm or shorter, and still more preferably 150 mm or shorter. When the distance from the nozzles to the collection surface is this upper limit or shorter, the fibers can be deposited densely. This should increase the water pressure resistance. In the meltblowing, the molten fibers are cooled and deposited. This prevents the molten fibers from being unified, thereby capable of improving the water pressure resistance. Because of the above, it is preferable that the fiber is cold by the time of fiber deposition. The distance from the nozzles to the collection surface is preferably 50 mm or longer, more preferably 80 mm or longer, and still more preferably 100 mm or longer.

The method of producing a meltblown non-woven fabric according to the invention preferably includes a heating step of heating the fiber after the spinning step and before the deposition step of depositing the fiber on the collection surface. From the viewpoint of just giving heat to the fiber without giving any disturbance such as a wind during the heating step, it is preferable to use an IR heater instead of a hot wind. In addition, from the viewpoint of preventing the nozzle-discharged fiber from being cooled and solidified in a spinning space, the fiber-heating position is located preferably 100 mm or more and preferably 200 mm or less below the nozzles. As such, unlike JP 2015-59294 A and WO 2012/014501 A, fibers immediately after spinning are not heated and a spinning space is instead preferably heated. Further, the fiber-heating position is located preferably 80 mm or more and more preferably 100 mm or more and preferably 200 mm or less and more preferably 180 mm or less in a direction perpendicular to the spinning line.

Collectively, in the method of producing a meltblown non-woven fabric according to the invention, the air stream temperature during the spinning step is set to 260°C or lower and the heat of fusion of thermoplastic resin composition as a component of the fiber is set to be larger than 5 mJ/mg but less than 94 mJ/mg. This makes it possible to obtain a meltblown non-woven fabric in which the rate of straight-running fibers in the CD direction is 35% or higher.

Examples

Next, Examples of the invention will be described. The invention, however, is not limited to them.

[Thermoplastic Resin Composition]

The resins used as raw materials for thermoplastic resin compositions in Examples 1 to 11 and Comparative Examples 1 to 4 are as follows:

resin 1: a polypropylene (Moplen (registered trademark) HP461Y, manufactured by Lyondellbasell, Inc.) with a heat of fusion of 98 mJ/mg, an MFR of 1300 g/10 min and a melting point of 160°C;
resin 2: a low crystalline polypropylene (L-MODU (registered trademark) S400, manufactured by Idemitsu Kosan Co., Ltd.) with a heat of fusion of 0.3 mJ/mg, an MFR of 2600 g/10 min, an Mw of 45000, and an Mw/Mn of 2, which is a low stereoregular polypropylene; and
resin 3: a polypropylene-based elastomer (Tafthren (registered trademark) H5002, manufactured by Sumitomo Chemical Industry Company Limited) with a heat of fusion of 6 mJ/mg, an MFR of 10 g/10 min, a melting point of 135°C, an Mw of 230000, and an Mw/Mn of 1.8, which is an amorphous propylene-(1-butene) copolymer.

The meltblown non-woven fabrics used in Comparative Examples 5 and 6 are as follows:

non-woven fabric 1: a meltblown non-woven fabric (PC0009) manufactured by Kuraray Kuraflex CO., LTD.; and
non-woven fabric 2: a meltblown non-woven fabric (P010SW-00X) manufactured by TAPYRUS CO., LTD.

[Evaluation]

Next, the following shows protocols for measuring various physical properties of meltblown non-woven fabrics according to Examples and Comparative Examples. Table 2 lists the results of measuring the various physical properties.

(1) Heat of Fusion of Thermoplastic Resin Composition

The heat of fusion of each thermoplastic resin composition was measured by differential scanning calorimetry (DSC). A DSC curve was plotted while the temperature was raised from 30°C at a rate of 20°C/min, and the heat amount at an endothermic peak seen between 100 and 200°C was defined as a heat of fusion.

(2) Basis Weight

The basis weight of each meltblown non-woven fabric was determined by excising three 50-mm square measurement pieces from a center portion of the meltblown non-woven fabric, measuring the mass (g) of each measurement piece, dividing the mass by the area ($m^2$) of the measurement piece, and taking the arithmetic means of the three pieces to yield the basis weight.

(3) Filling Factor

The filling factor of each meltblown non-woven fabric can be calculated using the following Equation (2). The thickness of each meltblown non-woven fabric was measured by using a laser displacement meter, manufactured by OMRON Corporation, while a load was imposed so as to apply a pressure of 4 kPa. Each thickness measurement was repeated five times and was averaged to yield the thickness of each meltblown non-woven fabric. Note that the fiber density was measured by the procedure described in JIS K 7112, specifically, pycnometry.
[Expression 3]

$$\text{Filling factor (\%)} = [(\text{Basis weight } [\text{g/m}^2] / 10000) / (\text{Thickness } [\mu\text{m}] / 10000) \times \text{Fiber density } [\text{g/m}^3] \times 100] \qquad (2)$$

(4) Formation Index

The formation index of each meltblown non-woven fabric was calculated using a formation tester (FMT-MIII) manufactured by NOMURA SHOJI CO., LTD. Specifically, each meltblown non-woven fabric sample was placed on a sample table and a CCD camera was used to capture a transmission image when one surface side of the sample was irradiated with light while the height of the CCD camera was set to 26 cm, the effective size was set to 10 cm × 10 cm, and the moving average pixel was set to 1. The effective size 10 cm × 10 cm was resolved into 320 × 230 pixels. Then, the intensity of light given to each pixel was measured and the transmittance T for each pixel was calculated using the following Equation (3):

[Expression 4]

$$\text{Transmittance T (\%)} = [(V_T - V_R)/(V_{100} - V_0)] \times 100 \quad \dots \quad (3)$$

where $V_T$ is a transmitted light volume when lighting is on (there is a sample); $V_R$ is a transmitted light volume when lighting is off (there is a sample); $V_{100}$ is a transmitted light volume when lighting is on (there is no sample); and $V_0$ is a transmitted light volume when lighting is off (there is no sample).

The resulting transmittance T was used to calculate absorbance E by using the following Equation (4):

[Expression 5]

$$\text{Absorbance E} = 2 - \log T \quad \dots \quad (4)$$

The resulting absorbance E was used to calculate a formation index by using the following Equation (5):

[Expression 6] Formation index = Coefficient of variation of absorbance E × 1000 = (Standard deviation ($\sigma$) of absorbance E/ Average of absorbance E) × 100          (5)

Three test pieces were measured and the measured values were averaged to yield the formation index of the sample.

Note that when the size of a sample is small and the dimension of 10 cm × 10 cm cannot be obtained as an effective size, the sample may be placed on the center of a test table and measured by setting the effective size to be less than the dimension of the sample but the area to be as large as possible, so as to calculate the formation index of the sample.

(5) Average Fiber Diameter

To measure the average fiber diameter, five small piece samples were randomly collected from each meltblown non-woven fabric. Next, a desktop scanning electron microscopy (JCM-6000 Plus), manufactured by JEOL Ltd., was used to capture a magnified SEM photo image having from 20 to 60 fibers seen in the visual field. The fiber diameters of all the fibers in the visual field were each measured once, the measured values were averaged, and the average was rounded off to the 10 nm place of the average to yield the fiber diameter of the small piece samples. The fiber diameters of five small piece samples were likewise measured and averaged to yield the average fiber diameter of each meltblown non-woven fabric.

(6) Rate of Straight-running Fibers

Regarding the meltblown non-woven fabrics according to Examples and Comparative Examples, the above-described way was used to calculate the rates of straight-running fibers in the first direction and the second direction. Here, because the MD direction and the CD direction of each non-woven fabric in Examples and Comparative Examples had been known, the MD direction was set to the first direction and the CD direction was set to the second direction.

(7) Water Pressure Resistance

The water pressure resistance was measured in accordance with the A method (low water pressure method) in the water penetration test (hydrostatic test) of JIS L1092-1998. During the water penetration test, a nylon mesh sheet (DO-ML-20 with a pore size of 133 um and a thickness of 121 um; manufactured by KURABO INDUSTRIES LTD.) was layered on each test piece, which was then measured. Note that when the dimension of the test piece does not conform to the specifics, the water pressure resistance can be measured by substantially the same method whereas an apparatus was assembled so as to reduce the measurement area such that water is applied to the test piece having an area capable of being collected.

Ten test pieces were measured and averaged to yield the water pressure resistance of each meltblown non-woven fabric according to Examples and Comparative Examples.

(8) Post-deformation Water Pressure Resistance

The CD direction and the MD direction during production of each meltblown non-woven fabric according to Examples and Comparative Examples were set to the lengthwise direction and the widthwise direction, respectively. Then, the meltblown non-woven fabric was excised into the size of a length of 130 mm or longer × a width of 150 mm. Next, chucks (in a tensile tester AG-IS, manufactured by Shimadzu Corporation) were used for immobilization such that the distance between the chucks in the lengthwise direction was set to 130 mm. Next, each meltblown non-woven fabric was drawn (at a tensile rate of 1 cm/5 sec) such that the distance between the chucks in the lengthwise direction reached 150 mm, and the meltblown non-woven fabric was held for 5 sec and then deformed in the CD direction. After that, the chucks were released and the post-deformation water pressure resistance was measured, on a center portion of the deformed meltblown non-woven fabric, in substantially the same manner as in the water pressure resistance measurement of the above (7).

(9) Water Pressure Resistance Retention Rate

The water pressure resistance retention rate was calculated using the following Equation (6):
[Expression 7]

$$\text{Water pressure resistance retention rate (\%)}$$
$$= (\text{Post-deformation water pressure resistance}/$$
$$\text{Pre-deformation water pressure resistance}) \times 100 \ldots (6)$$

[Example 1]

Resin 1 and resin 2 were mixed at a blending ratio: resin 1/resin 2 = 97/3 to prepare a thermoplastic resin composition. The thermoplastic resin composition was used to produce a meltblown non-woven fabric according to Example 1.
The production conditions for the meltblown non-woven fabric according to Example 1 were as follows. Table 1 lists the production conditions.

Resin temperature (the temperature at the time of discharging from the nozzles): 270°C
Single hole discharge amount: 0.20 g/min/hole
Hot wind flow volume: 300 Nm$^3$
Hot wind blowing width: 400 mm
Hot wind temperature (the temperature of air stream during the spinning step): 200°C
Nozzle diameter: 0.15 mm; nozzle length: 3 mm; nozzle pitch: 0.85 mm
Distance from the nozzles to the collection surface: 300 mm.

Here, the above protocols (1) to (9) were used to measure the various physical properties of the meltblown non-woven fabric according to Example 1. Table 2 shows the results.

[Examples 2 to 11 and Comparative Examples 1 to 4]

Meltblown non-woven fabrics according to Examples 2 to 11 and Comparative Examples 1 to 4 were manufactured in the same manner as in Example 1 except that the kinds of resins, the blending ratio, and the production conditions were changed as designated in Table 1. Note that the production conditions not listed in Table 1 are the same as in Example 1. Here, like Example 1, various physical properties of meltblown non-woven fabrics according to Examples 2 to 11 and Comparative Examples 1 to 4 were measured. Table 2 shows the results.

[Example 12]

While the center of a short-wavelength infrared heater (model IRMA 900/160) manufactured by Heraeus, Inc.,

was located at a position 150 mm below the nozzles and 120 mm farther apart from and perpendicular to the spinning line, resin 1 was used and heated at an output of 100% (total output of 9000 W). A meltblown non-woven fabric according to Example 12 was produced in the same manner as in Example 1 except for the above production condition. In addition, like Example 1, various physical properties of the resulting meltblown non-woven fabric were measured. Table 2 shows the results.

[Comparative Examples 5 and 6]

Various physical properties of meltblown non-woven fabrics according to Comparative Examples 5 and 6 were measured like Example 1. Table 2 shows the results.

[Table 1]

| | Resin | Blending ratio | Hot wind temperature (°C) | Distance from nozzles to a collection surface (mm) |
|---|---|---|---|---|
| Example 1 | Resin 1/Resin 2 | 97/3 | 200 | 300 |
| Example 2 | Resin 1/Resin 2 | 80/20 | 200 | 300 |
| Example 3 | Resin 1/Resin 2 | 50/50 | 200 | 300 |
| Example 4 | Resin 1/Resin 2 | 30/70 | 200 | 300 |
| Example 5 | Resin 1/Resin 2 | 10/90 | 200 | 300 |
| Example 6 | Resin 1/Resin 2 | 97/3 | 235 | 300 |
| Example 7 | Resin 1/Resin 2 | 50/50 | 235 | 300 |
| Example 8 | Resin 1/Resin 2 | 97/3 | 200 | 150 |
| Example 9 | Resin 1/Resin 2 | 50/50 | 200 | 150 |
| Example 10 | Resin 1/Resin 2 | 30/70 | 200 | 150 |
| Example 11 | Resin 1/Resin 2 | 85/15 | 260 | 300 |
| Example 12 | Resin 1 | 100 | 200 | 300 |
| Comparative Example 1 | Resin 1/Resin 2 | 97/3 | 270 | 300 |
| Comparative Example 2 | Resin 1/Resin 2 | 50/50 | 270 | 300 |
| Comparative Example 3 | Resin 1/Resin 2 | 5/95 | 200 | 300 |
| Comparative Example 4 | Resin 1/Resin 3 | 70/30 | 260 | 300 |
| Comparative Example 5 | Non-woven fabric 1 | - | - | - |
| Comparative Example 6 | Non-woven fabric 2 | - | - | - |

[Table 2]

| | Heat of fusion (DSC) | Filling factor | Formation index | Average fiber diameter | Rate of straight-running fibers (%) | | A/B | Water pressure resistance | Post-deformation water pressure resistance | Water pressure resistance retention rate |
|---|---|---|---|---|---|---|---|---|---|---|
| | | | | | First direction (A) | Second direction (B) | | | | |
| | mJ/mg | % | | μm | | | | mmH$_2$O | mmH$_2$O | % |
| Example 1 | 91 | 5.4 | 184 | 2 | 78 | 45 | 1.7 | 520 | 460 | 88 |
| Example 2 | 75 | 5.6 | 196 | 2 | 76 | 48 | 1.6 | 550 | 520 | 95 |
| Example 3 | 48 | 6.9 | 158 | 1.8 | 76 | 45 | 1.7 | 600 | 580 | 97 |
| Example 4 | 29 | 8.8 | 150 | 1.6 | 79 | 40 | 2.0 | 760 | 700 | 92 |
| Example 5 | 8 | 6.4 | 210 | 1 | 76 | 44 | 1.7 | 610 | 600 | 98 |
| Example 6 | 91 | 5.3 | 180 | 1.1 | 78 | 45 | 1.7 | 720 | 720 | 100 |
| Example 7 | 46 | 3.7 | 177 | 1 | 78 | 38 | 2.1 | 720 | 640 | 89 |
| Example 8 | 91 | 6.8 | 210 | 3.2 | 75 | 36 | 2.1 | 610 | 610 | 100 |
| Example 9 | 47 | 8.7 | 170 | 3 | 72 | 42 | 1.7 | 840 | 830 | 99 |
| Example 10 | 29 | 8.8 | 254 | 2 | 75 | 40 | 1.9 | 800 | 780 | 98 |
| Example 11 | 91 | 2.7 | 341 | 3.6 | 79 | 44 | 1.8 | 150 | 150 | 100 |
| Example 12 | 98 | 5.3 | 190 | 2.1 | 75 | 42 | 1.8 | 550 | 550 | 100 |
| Comparative Example 1 | 90 | 3.5 | 206 | 0.8 | 87 | 20 | 4.4 | 830 | 100> | Less than 13 |
| Comparative Example 2 | 50 | 4.4 | 329 | 0.9 | 88 | 32 | 2.8 | 690 | 100> | Less than 15 |
| Comparative Example 3 | 5 | 6.7 | 233 | 1 | 85 | 24 | 3.5 | 100> | 100> | - |
| Comparative Example 4 | 80 | 2.5 | 500 | 7.8 | 84 | 38 | 2.2 | 100> | 100> | - |
| Comparative Example 5 | 94 | 5.4 | 229 | 1 | 87 | 22 | 4.0 | 440 | 100> | Less than 23 |
| Comparative Example 6 | 94 | 3.4 | 253 | 4.2 | 82 | 34 | 2.4 | 200 | 100> | Less than 50 |

As illustrated in Table 2, the meltblown non-woven fabrics according to Examples 1 to 12 had an average fiber diameter of 4 um or less and the rate of straight-running fibers in each of a first direction having the highest rate of straight-running fibers and a second direction orthogonal to the first direction was 35% or higher. Alternatively, the average fiber diameter is 4 um or less and the ratio of the rate of straight-running fibers in the first direction to the rate of straight-running fibers in the second direction of a plane of the meltblown non-woven fabric was 2.5 or less. This has demonstrated that the water pressure resistance retention rate was high and a decrease in the water pressure resistance due to deformation was suppressed.

## Claims

1. A meltblown non-woven fabric having an average fiber diameter of from 0.1 um to 4 um, the average fiber diameter being measured according to the method of the description, comprising
   a first direction that is along a plane of the meltblown non-woven fabric and has a highest rate of straight-running fibers and a second direction orthogonal to the first direction, wherein a heat of fusion of the fibers is larger than 5 mJ/mg but less than 94 mJ/mg, the heat of fusion being measured according to the method of the description, the meltblown non-woven fabric satisfying one or more conditions selected from the following:

   having a rate of straight-running fibers of 35% or higher in each of the first direction and the second direction; and
   having a water pressure resistance of the meltblown non-woven fabric of from 100 mm $H_2O$ to 10000 mm $H_2O$, the water pressure resistance being measured according to the method of the description, and having a water pressure resistance retention rate of 85% or higher when the meltblown non-woven fabric is deformed in the second direction, the water pressure resistance retention rate being measured according to the method of the description,
   **characterized in that** the meltblown non-woven fabric satisfies the condition having a ratio of the rate of straight-running fibers in the first direction to the rate of straight-running fibers in the second direction (the rate of straight-running fibers in the first direction/the rate of straight-running fibers in the second direction) of from 1 to 2.5;
   the meltblown non-woven fabric being obtainable by

   a spinning step of discharging a molten thermoplastic resin composition from a nozzle to produce a fiber by using an air stream,
   a temperature of the air stream being a melting point or higher of the thermoplastic resin composition, wherein the air stream temperature is 260°C or less,
   wherein the first direction and the second direction and the straight-running rates are determined through the following steps (a) to (g):

   (a) capturing an SEM image of the meltblown non-woven fabric at an observation position of a center portion thereof using a desktop scanning electron microscopy (JCM-6000 Plus, manufactured by JEOL Ltd.) at an observation magnification = 3000/an average fiber diameter (um), and setting a long-side direction of the SEM image to 0 degrees, provided that when a non-woven fabric to be measured has a substantially rectangular shape, the SEM image is obtained such that a longitudinal direction of the non-woven fabric is set to 0 degrees and the long-side direction of the SEM image is parallel to the 0 degrees direction;
   (b) capturing an SEM image when a visual field is rotated $\theta$ degrees and capturing X pieces of the SEM images at from 0 degrees to (180 - $\theta$) degrees when the visual field is further rotated by every $\theta$ degrees, where the number X of pieces = (180 degrees/$\theta$ degrees) - 1;
   (c) performing manipulations in steps (a) and (b) at 7 observation positions other than the observation position in step (a) to obtain the SEM images at X pieces $\times$ 8 positions, provided that the 8 observation positions are located within an area of 40 mm $\times$ 20 mm at the center portion of the meltblown non-woven fabric and are apart from one another by 10 mm;
   (d) calculating, using the following Equation (1), a rate of straight-running fibers at each angle from the 0 degrees to the (180 - $\theta$) degrees with respect to the respective SEM images obtained through steps (a) to (c), and rounding the rates off to closest whole numbers, and averaging the rates at the 8 positions to yield a straight-running rate at each angle;
   (e) setting an angle with a largest straight-running rate among the straight-running rates calculated in step (d) for each angle to a first direction and setting the straight-running rate with the largest value to a rate of straight-running fibers in the first direction;
   (f) setting a direction orthogonal to the first direction to a second direction; and

(g) capturing SEM images, a long-side direction of which is parallel to the second direction, at the 8 observation positions, calculating respective rates of straight-running fibers in the second direction by using the following Equation (1), averaging the rates at the 8 positions and rounding the average off to a closest whole number:

[Expression 1]

$$\mathtt{Straight\text{-}running\ rate\ (\%)=\ (N(0)\times2/(N(1)+N(2)))\times100\ \ldots\ (1)}$$

where respective N(0), N(1), and N(2) represent the following, wherein

N(0) is the number of fibers that continuously extend from one end to another end of each SEM image in the long-side direction;
N(1) is the number of fibers that intersect the one end in the long-side direction; and
N(2) is the number of fibers that intersect the other end in the long-side direction.

2. The meltblown non-woven fabric according to claim 1, wherein the water pressure resistance is from 100 mm $H_2O$ to 10000 mm $H_2O$.

3. The meltblown non-woven fabric according to of claim 1 or 2, wherein the water pressure resistance retention rate when the meltblown non-woven fabric is deformed in the second direction is 85% or higher.

4. The meltblown non-woven fabric according to any one of claims 1 to 3, wherein a filling factor thereof is from 3% to 30%, the filling factor being measured according to the method of the description.

5. The meltblown non-woven fabric according to any one of claims 1 to 4, wherein a formation index thereof is from 30 to 300, the formation index being measured according to the method of the description.

6. A leakage prevention sheet comprising the meltblown non-woven fabric according to any one of claims 1 to 5.

7. An absorption article comprising:

a liquid permeating surface sheet that is arranged on a skin facing surface side;
a liquid leakage-preventing other surface sheet that is arranged on a skin non-facing surface side; and
an absorbent that is interposed between the sheets, wherein
the other surface sheet is the leakage prevention sheet according to claim 6.

8. A method of producing a meltblown non-woven fabric according to claim 1 having an average fiber diameter of 4 um or less, the average fiber diameter being measured according to the method of the description, comprising

a spinning step of discharging a molten thermoplastic resin composition from a nozzle to produce a fiber by using an air stream,
a temperature of the air stream being a melting point or higher of the thermoplastic resin composition, wherein the air stream temperature is 260°C or less,
the thermoplastic resin composition having a heat of fusion of larger than 5 mJ/mg but less than 94 mJ/mg, the heat of fusion being measured according to the method of the description.

9. The meltblown non-woven fabric production method according to claim 8, wherein the thermoplastic resin composition comprises two or more different polyolefins having distinct heats of fusion.

10. The meltblown non-woven fabric production method according to claim 8 or 9, further comprising

a deposition step of depositing the fiber obtained in the spinning step on a collection surface, wherein
a distance from the nozzle to the collection surface is 400 mm or shorter.

**Patentansprüche**

1. Schmelzgeblasener Vliesstoff mit einem durchschnittlichen Faserdurchmesser von 0,1 μm bis 4 μm, wobei der durchschnittliche Faserdurchmesser gemäß dem Verfahren der Beschreibung gemessen wird, der aufweist:

eine erste Richtung, die entlang einer Ebene des schmelzgeblasenen Vliesstoffs verläuft und eine höchste Rate an gerade verlaufenden Fasern aufweist, und eine zweite Richtung orthogonal zur ersten Richtung, wobei eine Schmelzwärme der Fasern größer als 5 mJ/mg, aber kleiner als 94 mJ/mg ist, wobei die Schmelzwärme gemäß dem Verfahren der Beschreibung gemessen wird, wobei der schmelzgeblasene Vliesstoff eine oder mehrere Bedingungen erfüllt, die aus den folgenden ausgewählt sind:

mit einer Rate an gerade verlaufenden Fasern von 35 % oder mehr in jeder der ersten und zweiten Richtung; und mit einer Wasserdruckbeständigkeit des schmelzgeblasenen Vliesstoffs von 100 mm $H_2O$ bis 10000 mm $H_2O$, wobei die Wasserdruckbeständigkeit gemäß dem Verfahren der Beschreibung gemessen wird, und mit einer Wasserdruckbeständigkeits-Retentionsrate von 85 % oder höher, wenn der schmelzgeblasene Vliesstoff in der zweiten Richtung verformt wird, wobei die Wasserdruckbeständigkeits-Retentionsrate gemäß dem Verfahren der Beschreibung gemessen wird,

**dadurch gekennzeichnet, dass** der schmelzgeblasene Vliesstoff die Bedingung erfüllt, dass ein Verhältnis der Rate der gerade verlaufenden Fasern in der ersten Richtung zur Rate der gerade verlaufenden Fasern in der zweiten Richtung (die Rate der gerade verlaufenden Fasern in der ersten Richtung/die Rate der gerade verlaufenden Fasern in der zweiten Richtung) 1 bis 2,5 beträgt;

wobei der schmelzgeblasene Vliesstoff erhalten werden kann durch

einen Spinnschritt, bei dem eine geschmolzene thermoplastische Harzzusammensetzung aus einer Düse ausgestoßen wird, um eine Faser unter Verwendung eines Luftstroms herzustellen,

wobei eine Temperatur des Luftstroms dem Schmelzpunkt oder höher der thermoplastischen Harzzusammensetzung entspricht, wobei die Temperatur des Luftstroms 260°C oder weniger beträgt,

wobei die erste Richtung und die zweite Richtung und die Geradeauslaufraten durch die folgenden Schritte (a) bis (g) bestimmt werden:

(a) Aufnehmen eines REM-Bildes des schmelzgeblasenen Vliesstoffs an einer Beobachtungsposition eines mittleren Abschnitts davon unter Verwendung eines Tisch-Rasterelektronenmikroskops (JCM-6000 Plus, hergestellt von JEOL Ltd.) bei einer Beobachtungsvergrößerung = 3000/einem durchschnittlichen Faserdurchmesser (μm) und Einstellen einer Längsseitenrichtung des REM-Bildes auf 0 Grad, vorausgesetzt, dass, wenn ein zu messender Vliesstoff eine im Wesentlichen rechteckige Form hat, das REM-Bild so erhalten wird, dass eine Längsrichtung des Vliesstoffes auf 0 Grad eingestellt ist und die Längsseitenrichtung des REM-Bildes parallel zu der 0-Grad-Richtung ist;

(b) Aufnehmen eines REM-Bildes, wenn ein Sichtfeld um θ Grad gedreht wird, und Aufnehmen von X Teilen der REM-Bilder bei 0 Grad bis (180 - θ) Grad, wenn das Sichtfeld weiter um jeweils θ Grad gedreht wird, wobei die Anzahl X der Teile = (180 Grad/θ Grad) - 1 ist;

(c) Durchführen von Manipulationen in den Schritten (a) und (b) an 7 anderen Beobachtungspositionen als der Beobachtungsposition in Schritt (a), um die REM-Bilder an X Teilen × 8 Positionen zu erhalten, mit der Maßgabe, dass die 8 Beobachtungspositionen innerhalb eines Bereichs von 40 mm × 20 mm am Mittelabschnitt des schmelzgeblasenen Vliesstoffs liegen und um 10 mm voneinander entfernt sind;

(d) Berechnen, unter Verwendung der folgenden Gleichung (1), einer Rate von gerade verlaufenden Fasern bei jedem Winkel von 0 Grad bis (180 - θ) Grad in Bezug auf die jeweiligen REM-Bilder, die durch die Schritte (a) bis (c) erhalten wurden, und Runden der Raten auf die nächstliegenden ganzen Zahlen und Mitteln der Raten an den 8 Positionen, um eine gerade verlaufende Rate bei jedem Winkel zu erhalten;

(e) Einstellen eines Winkels mit einer größten Geradeauslaufrate unter den in Schritt (d) für jeden Winkel berechneten Geradeauslaufraten auf eine erste Richtung und Einstellen der Geradeauslaufrate mit dem größten Wert auf eine Rate von gerade verlaufenden Fasern in der ersten Richtung;

(f) Einstellen einer Richtung orthogonal zur ersten Richtung auf eine zweite Richtung; und

(g) Aufnehmen von REM-Bildern, von denen eine Längsseitenrichtung parallel zu der zweiten Richtung ist, an den 8 Beobachtungspositionen, Berechnen der jeweiligen Raten von gerade verlaufenden Fasern in der zweiten Richtung unter Verwendung der folgenden Gleichung (1), Mitteln der Raten an den 8 Positionen und Runden des Mittelwerts auf eine nächste ganze Zahl:

[Gleichung 1]

$$\text{Geradeauslaufrate (\%)} = (N(0) \times 2/(N(1) + N(2))) \times 100 \quad \dots \quad (1)$$

wobei N(0), N(1) und N(2) jeweils Folgendes darstellen, wobei

N(0) die Anzahl der Fasern ist, die sich in jedem REM-Bild in der Längsseitenrichtung kontinuierlich von einem Ende zum anderen Ende erstrecken;
N(1) die Anzahl der Fasern ist, die das eine Ende in der Längsseitenrichtung schneiden; und
N(2) die Anzahl der Fasern ist, die das andere Ende in der Längsseitenrichtung schneiden.

2. Schmelzgeblasener Vliesstoff nach Anspruch 1, wobei die Wasserdruckbeständigkeit zwischen 100 mm $H_2O$ und 10000 mm $H_2O$ liegt.

3. Schmelzgeblasener Vliesstoff nach Anspruch 1 oder 2, wobei die Wasserdruckbeständigkeits-Retentionsrate 85 % oder mehr beträgt, wenn der schmelzgeblasene Vliesstoff in der zweiten Richtung verformt wird.

4. Schmelzgeblasener Vliesstoff nach einem der Ansprüche 1 bis 3, wobei ein Füllfaktor zwischen 3 % und 30 % liegt, wobei der Füllfaktor gemäß dem Verfahren der Beschreibung gemessen wird.

5. Schmelzgeblasener Vliesstoff nach einem der Ansprüche 1 bis 4, wobei ein Formationsindex zwischen 30 und 300 liegt, wobei der Formationsindex gemäß dem Verfahren der Beschreibung gemessen wird.

6. Das Auslaufen verhindernde Lage, die den schmelzgeblasenen Vliesstoff nach einem der Ansprüche 1 bis 5 aufweist.

7. Absorptionsartikel, der aufweist:

eine flüssigkeitsdurchlässige Oberflächenlage, die auf einer der Haut zugewandten Oberflächenseite angeordnet ist;
eine das Auslaufen von Flüssigkeit verhindernde andere Oberflächenlage, die auf einer der Haut nicht zugewandten Oberflächenseite angeordnet ist; und
ein Absorptionsmittel, das zwischen den Lagen angeordnet ist, wobei die andere Oberflächenlage die das Auslaufen verhindernde Lage nach Anspruch 6 ist.

8. Verfahren zur Herstellung eines schmelzgeblasenen Vliesstoffs nach Anspruch 1 mit einem durchschnittlichen Faserdurchmesser von 4 μm oder weniger, wobei der durchschnittliche Faserdurchmesser gemäß dem Verfahren der Beschreibung gemessen wird, das aufweist:

einen Spinnschritt, bei dem eine geschmolzene thermoplastische Harzzusammensetzung aus einer Düse ausgestoßen wird, um eine Faser unter Verwendung eines Luftstroms herzustellen,
wobei eine Temperatur des Luftstroms dem Schmelzpunkt oder höher der thermoplastischen Harzzusammensetzung entspricht, wobei die Temperatur des Luftstroms 260°C oder weniger beträgt,
wobei die thermoplastische Harzzusammensetzung eine Schmelzwärme von größer als 5 mJ/mg, aber kleiner als 94 mJ/mg aufweist, wobei die Schmelzwärme gemäß dem Verfahren der Beschreibung gemessen wird.

9. Verfahren zur Herstellung eines schmelzgeblasenen Vliesstoffs nach Anspruch 8, wobei die thermoplastische Harzzusammensetzung zwei oder mehr verschiedene Polyolefine mit unterschiedlichen Schmelzwärmen aufweist.

10. Verfahren zur Herstellung eines schmelzgeblasenen Vliesstoffs nach Anspruch 8 oder 9, das ferner aufweist:

einen Ablagerungsschritt, bei dem die im Spinnschritt erhaltene Faser auf einer Sammelfläche abgelagert wird, wobei
ein Abstand zwischen der Düse und der Sammelfläche 400 mm oder weniger beträgt.

**Revendications**

1. Tissu non tissé obtenu par fusion-soufflage présentant un diamètre de fibre moyen de 0,1 μm à 4 μm, le diamètre de fibre moyen étant mesuré selon la méthode de la description, comprenant
une première direction qui est le long d'un plan du tissu non tissé obtenu par fusion-soufflage et présente un taux le plus élevé de fibres s'étendant en ligne droite et une seconde direction orthogonale à la première direction, dans lequel une chaleur de fusion des fibres est supérieure à 5 mJ/mg mais inférieure à 94 mJ/mg, la chaleur de fusion étant mesurée selon la méthode de la description, le tissu non tissé obtenu par fusion-soufflage satisfaisant une ou plusieurs conditions choisies parmi ce qui suit :

présenter un taux de fibres s'étendant en ligne droite supérieur ou égal à 35 % dans chacune de la première direction et de la seconde direction ; et

présenter une résistance à la pression de l'eau du tissu non tissé obtenu par fusion-soufflage de 100 mm H$_2$O à 10 000 mm H$_2$O, la résistance à la pression de l'eau étant mesurée selon la méthode de la description, et présenter un taux de rétention de la résistance à la pression de l'eau supérieur ou égal à 85 % lorsque le tissu non tissé obtenu par fusion-soufflage est déformé dans la seconde direction, le taux de rétention de la résistance à la pression de l'eau étant mesuré selon la méthode de la description,

**caractérisé en ce que** le tissu non tissé obtenu par fusion-soufflage satisfait la condition de présenter un rapport du taux de fibres s'étendant en ligne droite dans la première direction sur le taux de fibres s'étendant en ligne droite dans la seconde direction (le taux de fibres s'étendant en ligne droite dans la première direction/le taux de fibres s'étendant en ligne droite dans la seconde direction) de 1 à 2,5 ;

le tissu non tissé obtenu par fusion-soufflage pouvant être obtenu par

une étape de filage consistant à décharger une composition de résine thermoplastique fondue depuis une buse pour produire une fibre à l'aide d'un flux d'air,

une température du flux d'air étant supérieure ou égale à un point de fusion de la composition de résine thermoplastique, dans lequel la température de flux d'air est inférieure ou égale à 260 °C,

dans lequel la première direction et la seconde direction et les taux de fibres s'étendant en ligne droite sont déterminés par les étapes (a) à (g) suivantes :

(a) la capture d'une image SEM du tissu non tissé obtenu par fusion-soufflage à une position d'observation d'une partie centrale de celui-ci à l'aide d'un microscope électronique à balayage de table (JCM-6000 Plus, fabriqué par JEOL Ltd.) à un grossissement d'observation = 3000/un diamètre de fibre moyen (pm), et la définition d'une direction de côté long de l'image SEM à 0 degré, à condition que, lorsqu'un tissu non tissé obtenu à mesurer présente une forme sensiblement rectangulaire, l'image SEM soit obtenue de sorte qu'une direction longitudinale du tissu non tissé soit définie à 0 degré et la direction de côté long de l'image SEM soit parallèle à la direction de 0 degré ;

(b) la capture d'une image SEM lorsqu'un champ visuel est tourné de θ degrés et la capture de X pièces des images SEM à un angle de 0 degré à (180 - θ) degrés lorsque le champ visuel est tourné davantage de θ degrés à la fois, où le nombre X de pièces = (180 degrés/θ degrés) - 1 ;

(c) la réalisation des manipulations des étapes (a) et (b) à 7 positions d'observation autres que la position d'observation de l'étape (a) pour obtenir les images SEM à X pièces x 8 positions, à condition que les 8 positions d'observation soient situées à l'intérieur d'une aire de 40 mm x 20 mm au niveau de la partie centrale du tissu non tissé obtenu par fusion-soufflage et soient espacées les unes des autres de 10 mm ;

(d) le calcul, en utilisant l'équation (1) suivante, d'un taux de fibres s'étendant en ligne droite à chaque angle dans la plage de 0 degré à (180 - θ) degrés par rapport aux images SEM respectives obtenues aux étapes (a) à (c), et l'arrondissement des taux aux nombres entiers les plus proches, et le moyennage des taux aux 8 positions pour donner un taux de fibres s'étendant en ligne droite à chaque angle ;

(e) la définition d'un angle avec un taux de fibres s'étendant en ligne droite le plus grand parmi les taux de fibres s'étendant en ligne droite calculés à l'étape (d) pour chaque angle à une première direction et la définition du taux de fibres s'étendant en ligne droite avec la plus grande valeur à un taux de fibres s'étendant en ligne droite dans la première direction ;

(f) la définition d'une direction orthogonale à la première direction à une seconde direction ; et

(g) la capture d'images SEM, dont une direction de côté long est parallèle à la seconde direction, aux 8 positions d'observation, le calcul de taux respectifs de fibres s'étendant en ligne droite dans la seconde direction à l'aide de l'équation (1) suivante, le moyennage des taux aux 8 positions et l'arrondissement de la moyenne à un nombre entier le plus proche :

[Equation 1] taux de fibres s'étendant en ligne droite (%) = (N(0) $\times$2/ (N(1) +N(2))) $\times$100       (1)

où N(0), N(1) et N(2) représentent respectivement ce qui suit :

N(0) est le nombre de fibres s'étendant continuellement d'une extrémité à une autre extrémité de chaque image SEM dans la direction de côté long ;
N(1) est le nombre de fibres en intersection avec l'extrémité dans la direction de côté long ; et
N(2) est le nombre de fibres en intersection avec l'autre extrémité dans la direction de côté long.

2. Tissu non tissé obtenu par fusion-soufflage selon la revendication 1, dans lequel la résistance à la pression de l'eau est de 100 mm $H_2O$ à 10 000 mm $H_2O$.

3. Tissu non tissé obtenu par fusion-soufflage selon la revendication 1 ou 2, dans lequel le taux de rétention de la résistance à la pression de l'eau lorsque le tissu non tissé obtenu par fusion-soufflage est déformé dans la seconde direction est supérieur ou égal à 85 %.

4. Tissu non tissé obtenu par fusion-soufflage selon l'une quelconque des revendications 1 à 3, dans lequel un facteur de remplissage de celui-ci est de 3 % à 30 %, le facteur de remplissage étant mesuré selon la méthode de la description.

5. Tissu non tissé obtenu par fusion-soufflage selon l'une quelconque des revendications 1 à 4, dans lequel un indice de formation de celui-ci est de 30 à 300, l'indice de formation étant mesuré selon la méthode de la description.

6. Feuille de prévention de fuite comprenant le tissu non tissé obtenu par fusion-soufflage selon l'une quelconque des revendications 1 à 5.

7. Article d'absorption comprenant :

une feuille de surface perméable au liquide qui est agencée sur un côté de surface faisant face à la peau ;
une autre feuille de surface de prévention de fuite de liquide qui est agencée sur un côté de surface ne faisant pas face à la peau ; et
un absorbant qui est interposé entre les feuilles, dans lequel
l'autre feuille de surface est la feuille de prévention de fuite selon la revendication 6.

8. Méthode de production d'un tissu non tissé obtenu par fusion-soufflage selon la revendication 1 présentant un diamètre de fibre moyen inférieur ou égal à 4 $\mu$m, le diamètre de fibre moyen étant mesuré selon la méthode de la description, comprenant

une étape de filage consistant à décharger une composition de résine thermoplastique fondue depuis une buse pour produire une fibre à l'aide d'un flux d'air,
une température du flux d'air étant supérieure ou égale à un point de fusion de la composition de résine thermoplastique, dans laquelle la température de flux d'air est inférieure ou égale à 260 °C,
la composition de résine thermoplastique présentant une chaleur de fusion supérieure à 5 mJ/mg mais inférieure à 94 mJ/mg, la chaleur de fusion étant mesurée selon la méthode de la description.

9. Méthode de production de tissu non tissé obtenu par fusion-soufflage selon la revendication 8, dans laquelle la composition de résine thermoplastique comprend deux polyoléfines différentes ou plus présentant des chaleurs de fusion distinctes.

10. Méthode de production de tissu non tissé obtenu par fusion-soufflage selon la revendication 8 ou 9, comprenant en outre

une étape de dépôt consistant à déposer la fibre obtenue à l'étape de filage sur une surface de collecte, dans laquelle
la distance de la buse à la surface de collecte est inférieure ou égale à 400 mm.

FIG. 1

SECOND DIRECTION

FIRST DIRECTION

EP 3 730 685 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012102398 A
- EP 2527508 A1
- EP 3255188 A1
- US 2013266874 A1
- JP 2015059294 A
- WO 2012014501 A